(19) Europäisches Patentamt
European Patent Office
Office européen des brevets

(11) EP 4 011 276 A1

(12) **EUROPEAN PATENT APPLICATION**
published in accordance with Art. 153(4) EPC

(43) Date of publication:
**15.06.2022 Bulletin 2022/24**

(21) Application number: **19941176.0**

(22) Date of filing: **09.08.2019**

(51) International Patent Classification (IPC):
**A61B 5/00** (2006.01)    **A61B 5/145** (2006.01)

(52) Cooperative Patent Classification (CPC):
**A61B 5/00; A61B 5/145**

(86) International application number:
**PCT/CN2019/100080**

(87) International publication number:
**WO 2021/026698 (18.02.2021 Gazette 2021/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Gimer Medical. Co., Ltd
New Taipei City 22175 (TW)**

(72) Inventors:
• **CHANG, Chi-Heng
New Taipei City, Taiwan 22175 (CN)**
• **WANG, Mei-Cing
New Taipei City, Taiwan 22175 (CN)**

(74) Representative: **Michalski Hüttermann & Partner
Patentanwälte mbB
Kaistraße 16A
40221 Düsseldorf (DE)**

(54) **INJECTION-TYPE TESTING DEVICE, TESTING SYSTEM, AND TESTING METHOD**

(57) An implantable inspecting device for inspecting an analyte in a liquid is provided, including a hollow carrier, a light source, a first polarizing member, a second polarizing member, and a sensor. The hollow carrier includes an accommodating space and at least one cavity portion communicated with the accommodating space. The light source, the first polarizing member, the second polarizing member, and the sensor are disposed in the accommodating space. The light from the light source passes through the first polarizing member, the liquid, and the second polarizing member to reach the sensor.

FIG. 2

EP 4 011 276 A1

## Description

## Technology Field

The application relates in general to an implantable inspecting device, and in particular, to an implantable inspecting device for inspecting an analyte in a liquid.

## Background

With the advancement of medical treatment, human life is getting longer, and the numbers of the patients with metabolic disorders are therefore increased. For example, diabetes is an important example of a metabolic disorder. Patients need to monitor their blood sugar levels, so as to prevent complications.

Currently, there are two common inspecting methods, one of which involves dripping blood on a test paper. Another involves the insertion of a probe into the human body to measure the amount of glucose in the blood or other bodily fluid. However, the results of the aforementioned measurement methods can easily become inaccurate due to contamination, and the inspecting methods are not convenient to inspect, the required information cannot be obtained at all times. Therefore, how to address the aforementioned problem has become an important issue.

## Summary

To address the deficiencies of conventional products, an embodiment of the disclosure provides an implantable inspecting device for inspecting an analyte in a liquid is provided, including a hollow carrier, a light source, a first polarizing member, a second polarizing member, and a sensor. The hollow carrier includes an accommodating space and at least one cavity portion communicated with the accommodating space. The light source, the first polarizing member, the second polarizing member, and the sensor are disposed in the accommodating space. The light from the light source passes through the first polarizing member, the liquid, and the second polarizing member to reach the sensor.

An inspecting system for inspecting an analyte in a liquid is also provided, including an implantable inspecting device and a reading device. The implantable inspecting device includes a hollow carrier, a light source, a first polarizing member, a second polarizing member, and a sensor. The hollow carrier includes an accommodating space and at least one cavity portion communicated with the accommodating space. The light source, the first polarizing member, the second polarizing member, and the sensor are disposed in the accommodating space. The light from the light source passes through the first polarizing member, the liquid, and the second polarizing member to reach the sensor. The wireless transmitting module is electrically connected to the sensor. When the reading device is adjacent to the inspecting device, the wireless transmitting module transmits inspected data from the sensor to the reading device in a wireless manner.

An inspecting method for an implantable inspecting device is also provided, including: providing the implantable inspecting device, wherein the implantable inspecting device includes a hollow carrier, a light source, a first polarizing member, a second polarizing member, and a sensor, the hollow carrier includes an accommodating space and at least one cavity portion communicated with the accommodating space, and the light source, the first polarizing member, the second polarizing member, and the sensor are disposed in the accommodating space; letting a liquid pass through the at least one cavity portion and enter the accommodating space; using the light source to provide a light, wherein the light passes through the first polarizing member, the liquid, and the second polarizing member to reach the sensor; and using the sensor to inspect the optical rotation and/or the light absorption of the light, so as to inspect the analyte.

An inspecting method for an implantable inspecting device is further provided, including: providing an implantable inspecting device, wherein the implantable inspecting device includes a hollow carrier, a light source, a first polarizing member, a second polarizing member, and a sensor, the hollow carrier includes an accommodating space and at least one cavity portion communicated with the accommodating space, and the light source, the first polarizing member, the second polarizing member, and the sensor are disposed in the accommodating space; using an injecting tool to implant the implantable inspecting device into the human body; proliferating the tissue of the human body to enclose at least a portion of an outer surface of the hollow carrier; letting the bodily fluid of the human body pass through the at least one cavity portion and enter the accommodating space; using the light source to provide a light, wherein the light passes through the first polarizing member, the bodily fluid of the human body, and the second polarizing member to reach the sensor; and using the sensor to inspect the optical rotation and/or the light absorption of the light, so as to inspect the analyte.

## Description of drawings

Fig. 1 is a schematic diagram of an inspecting system according to an embodiment of the invention.

Fig. 2 is an exploded-view diagram of an implantable inspecting device according to an embodiment of the invention.

Fig. 3 is a cross-sectional view of the implantable inspecting device according to an embodiment of the invention.

Fig. 4 is a schematic diagram of a hollow carrier according to an embodiment of the invention.

Fig. 5A is a schematic diagram of a hollow carrier according to another embodiment of the invention.

Fig. 5B is a schematic diagram of a hollow carrier according to another embodiment of the invention.

Fig. 5C is a schematic diagram of a hollow carrier according to another embodiment of the invention.

Fig. 5D is a schematic diagram of a hollow carrier according to another embodiment of the invention.

Fig. 5E is a schematic diagram of a hollow carrier according to another embodiment of the invention.

Fig. 6A is a schematic diagram of a hollow carrier according to another embodiment of the invention.

Fig. 6B is a schematic diagram of a hollow carrier according to another embodiment of the invention.

Fig. 6C is a schematic diagram of a hollow carrier according to another embodiment of the invention.

Fig. 7 is a schematic diagram of a membrane member according to another embodiment of the invention.

Fig. 8A is a schematic diagram of a data inspected by the implantable inspecting device according to an embodiment of the invention.

Fig. 8B is a schematic diagram of an implantable inspecting device according to another embodiment of the invention.

Fig. 9 is a schematic diagram of an implantable inspecting device according to another embodiment of the invention.

Fig. 10 is a flow chart of an inspecting method according to an embodiment of the invention.

The reference numerals in drawings are as below:

    100 hollow carrier
    101 inner wall
    110 accommodating space
    120 cavity portions
    200 light source
    300 first polarizing member
    400 second polarizing member
    500 sensor
    600 transmitting module

    700 membrane member
    710 retardant layer
    720 proliferation layer
    800 sealing member
    910 first light valve module
    920 second light valve module
    B human body
    D diplay panel
    L1, L2line
    P implantable inspecting device
    R reading device
    S main body
    S1~S8 step

## Detailed description of invention

The making and using of the embodiments of the implantable inspecting device, the inspecting system, and the inspecting method are discussed in detail below. It should be appreciated, however, that the embodiments provide many applicable inventive concepts that can be embodied in a wide variety of specific contexts. The specific embodiments discussed are merely illustrative of specific ways to make and use the embodiments, and do not limit the scope of the disclosure.

Unless defined otherwise, all technical and scientific terms used herein have the same meaning as commonly understood by one of ordinary skill in the art to which this invention belongs. It should be appreciated that each term, which is defined in a commonly used dictionary, should be interpreted as having a meaning conforming to the relative skills and the background or the context of the present disclosure, and should not be interpreted in an idealized or overly formal manner unless defined otherwise.

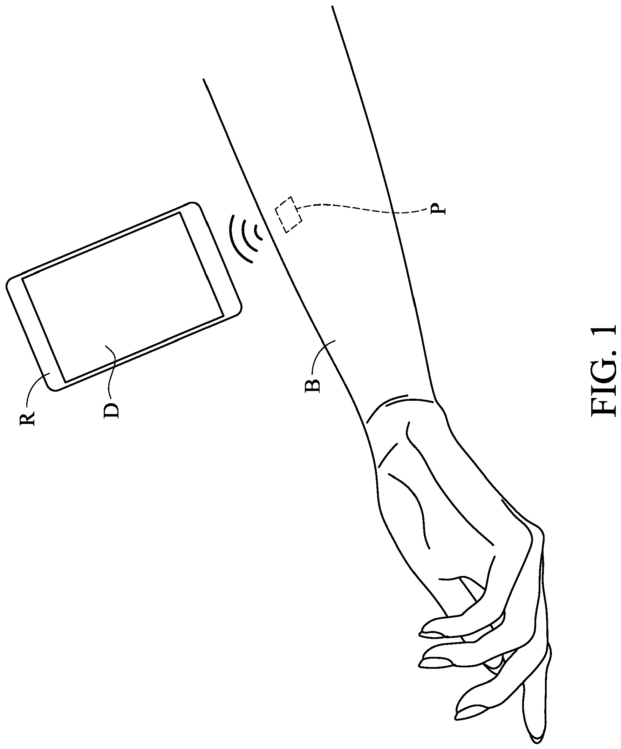

Referring to Fig. 1, in an embodiment of invention, an inspecting system includes an implantable inspecting device P and a reading device R, wherein the implantable inspecting device P can be implant into a subcutaneous tissue of a human body B to continuously inspect the concentration of an analyte (such as glucose, urate crystal, lactic acid, or etc.) in a bodily fluid of the human body B, or the presence or absence of the analyte in a bodily fluid of the human body B. The continuous measurement can be defined as a measurement every 0.5, 1, 3, 5, 10, 15, 20, 30, 40, 50, 60, 90, 120, or 180 minutes. For example, when the user desires to inspect the analyte in the bodily fluid, the reading device R can be attached to or close to the skin surface of the human body B where the implantable inspecting device P is embedded. At that time, the implantable inspecting device P can transmit the inspected data to the reading device R in a wireless manner, and the reading device R can display the aforementioned data on its display panel D. Therefore, it is convenient for the user to obtain the information of the analyte, and further understand his/her own body conditions.

Moreover, the reading device R can further include a wireless power supply. When the reading device R is used to read the data of the implantable inspecting device P, it can firstly provide power to the implantable inspecting device P to inspect the data and then read the data. The implantable inspecting device P can also have at least one battery, so that it can provide energy itself to the light source, the sensing member, and the wireless transmitting module to operate, and the analysis of the concentration of the analyte can be processed at all times.

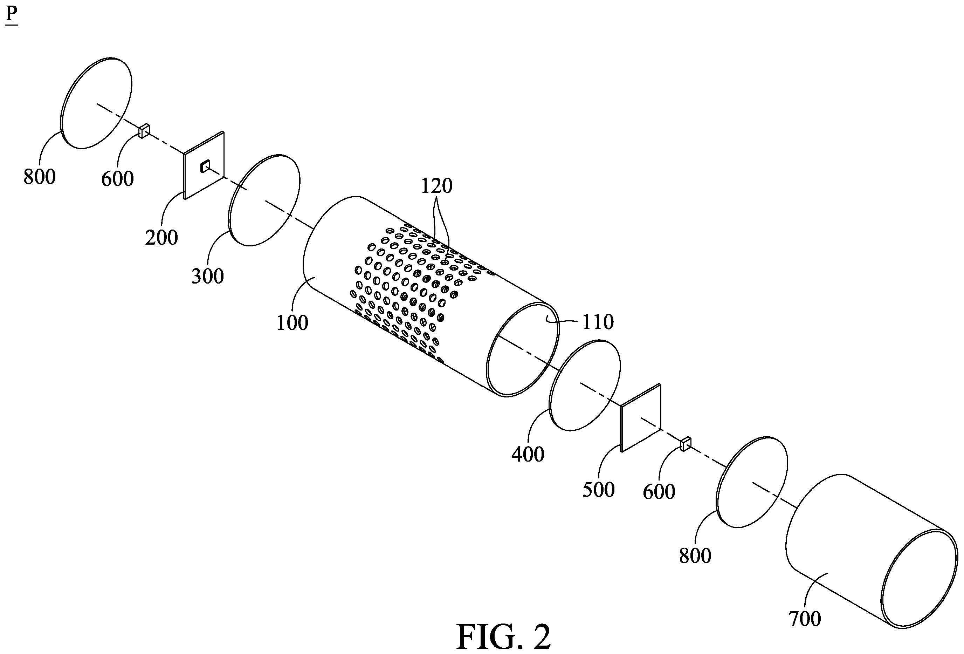

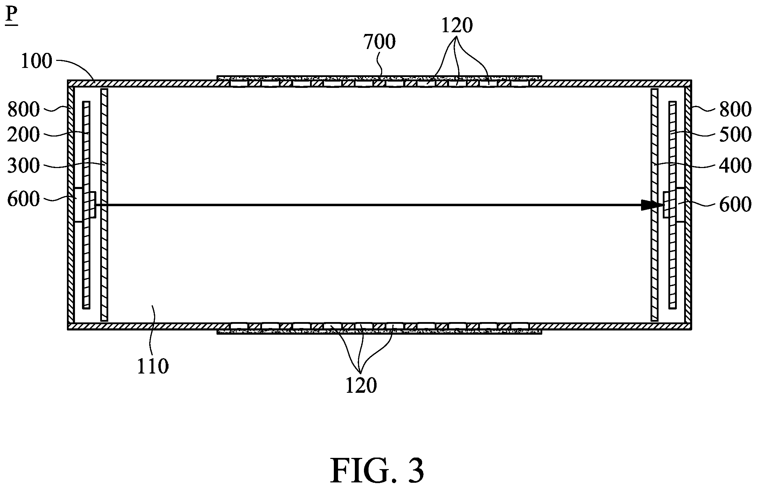

Fig. 2 is an exploded-view diagram of the implantable inspecting device P, and Fig. 3 is a cross-sectional view of the implantable inspecting device P. As shown in Figs. 2 and 3, the implantable inspecting device P primarily includes a hollow carrier 100, a light source 200, a first polarizing member 300, a second polarizing member 400, a sensor 500, at least one wireless transmitting module 600, a membrane member 700, and at least one sealing member 800.

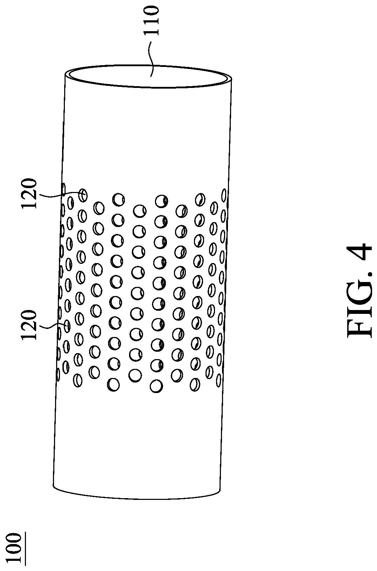

As shown in Figs. 3 and 4, the hollow carrier 100 substantially has a pillar structure, and includes an accommodating space 110 and at least one cavity portion 120. The light source 200, the first polarizing member 300, the second polarizing member 400, the sensor 500, and the wireless transmitting module 600 are accommodated in the accommodating space 110 of the hollow carrier 100. The cavity portion 120 communicates the accommodating space 110 with an external environment.

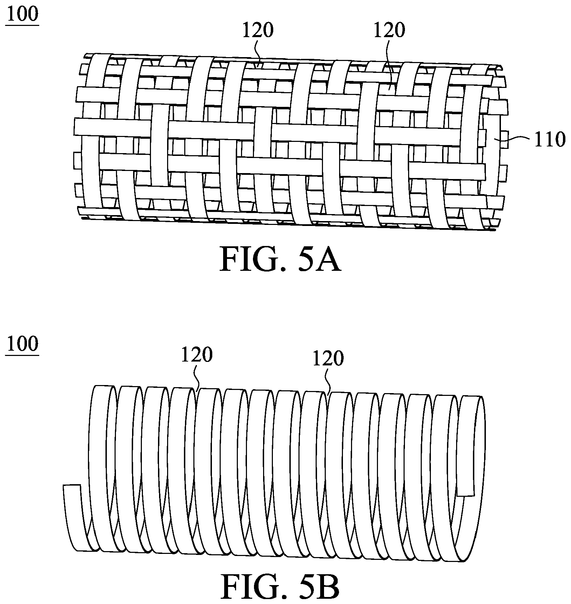

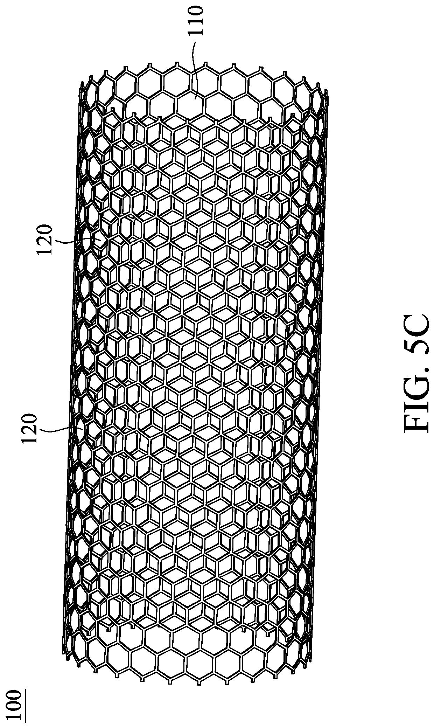

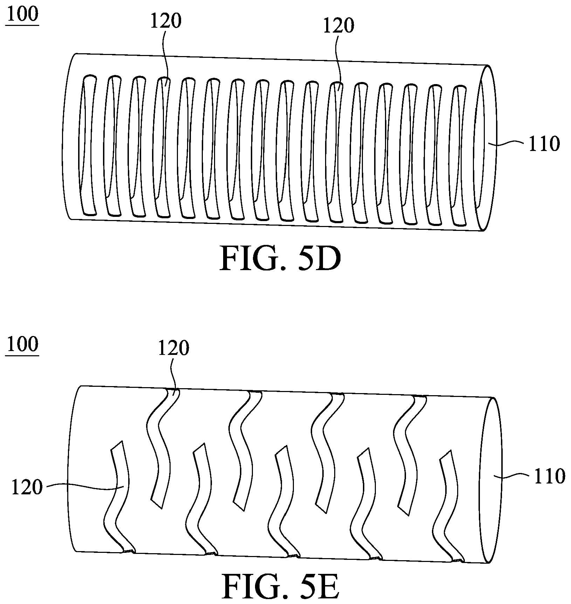

In this embodiment, each of the cavity portions 120 substantially has a circular hole structure, and the cavity portions 120 are separately arranged on the hollow carrier 100 at equal intervals or unequal intervals. The cavity portions 120 are arranged on the middle section of the hollow carrier 100 in this embodiment. The cavity portions 120 can also arranged on all sections of the hollow carrier 100. As shown in Figs. 5A-5E, in some embodiments, each of the cavity portions 120 can include a polygonal structure, a spiral structure, a longitudinal structure, or an irregular shape structure. It should be noted that, the total area of the cavity portions 120 take up 30% - 70% of the surface area of the space in which the hollow carrier 100 is disposed, so as to facilitate the liquid with the analyte entering. The liquid with the analyte can be the bodily fluid, and the bodily fluid primarily includes the extracellular fluid, especially the interstitial fluid. When the implanted hollow carrier 100 is positioned in the muscle tissue, the liquid flowing into or infiltrating into the accommodating space 110 of the hollow carrier 100 is the interstitial fluid in the muscle tissue, wherein the main component of the muscle tissue includes glucose, fatty acid, salt, and mineral (such as calcium, magnesium, and/or potassium).

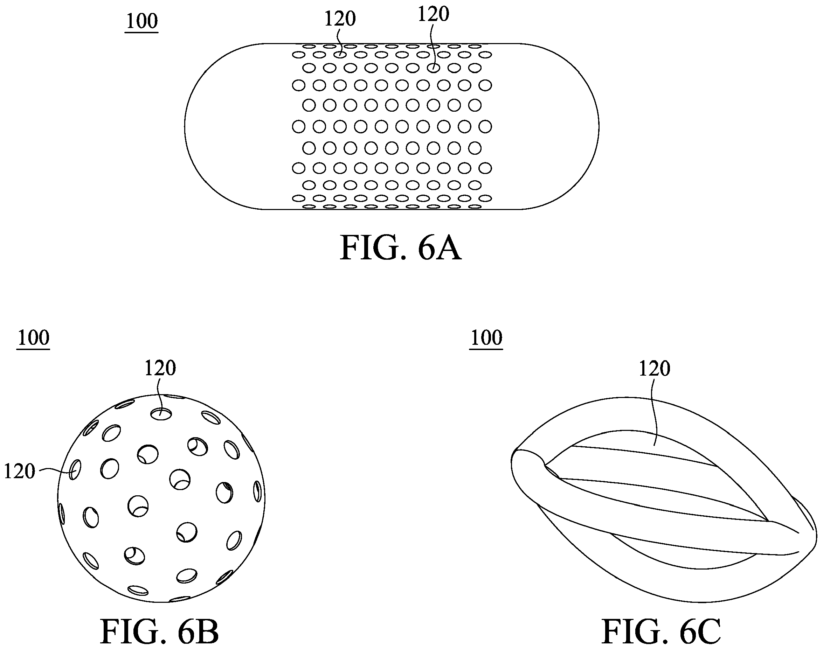

Referring to Figs. 6A-6C, in some embodiments, the appearance of the hollow carrier 100 can be adjusted as required. For example, it can be formed as a capsule-shaped structure, a ball structure, or a fusiform-shaped structure. The structure can be integrally formed in one piece or formed by assembly. The appearances of the hollow carrier 100 and the cavity portions 120 can be adjusted as required, and it is not limited to the appearance and the shape shown in the aforementioned figures.

Referring to Figs. 2 and 3, when the light source 200, the first polarizing member 300, the second polarizing member 400, the sensor 500, and the wireless transmitting module 600 are disposed in the accommodating space 110, the light source 200, the first polarizing member 300, the second polarizing member 400, and the sensor 500 are arranged in sequence along the longitudinal axis of the hollow carrier 100. In other words, the first polarizing member 300 and the second polarizing member 400 are disposed between the light source 200 and the sensor 500, and the first polarizing member 300 is disposed between the light source 200 and the second polarizing member 400. Moreover, the cavity portions 120 on the sidewall of the hollow carrier 100 are disposed between the first polarizing member 300 and the second polarizing member 400.

The light source 200 can include one or more light-emitting diodes (LED) or laser diodes disposed on a printed circuit board or a flexible board. The light source 200 can emit a light with a wavelength between the near infrared and the blue light, for example, the light with the wavelength ranged in 500nm-1000nm. Since the laser diode is coherent and highly directional, it can be a preferable choice. The first polarizing member 300 and the second polarizing member 400 can respectively be a polarizer and an analyzer. Preferably, the optical axis of the first polarizing member 300 and the optical axis of the second polarizing member 400 are orthogonal. When the liquid including the analyte enters the accommodating space 110, the light from the light source 200 can pass the first polarizing member 300, the liquid, and the second polarizing member 400 in sequence, and finally reach the sensor 500.

Since the first polarizing member 300 can polarize the light from the light source 200, and the liquid has the chiral material (i.e. the aforementioned analyte), so that the moving direction of the light is rotated. The sensor 500 can receive the light rotated by the chiral material, and obtain the concentration of the analyte, the presence or absence of the analyte, or whether the amount of the analyte is greater than a threshold by inspecting the optical rotation and/or the light absorption of the light.

For example, the concentration of the analyte can be obtained according to the following formula:

$$C = \frac{\theta}{[\alpha]_{\lambda.T}^{pH} \cdot L},$$

wherein C is the concentration of the analyte (g/100mL), θ is the optical rotation angle measured by the sensor

$$[\alpha]_{\lambda.T}^{pH}$$

500, $[\alpha]_{\lambda.T}^{pH}$ is the optical rotation angle (for example, the optical rotation angle of glucose is +52.7∘), and L is the optical path (decimeter). According to the aforementioned formula, it can be noted that the concentration of the analyte is proportional to the optical rotation angle. The optical rotation angle and the optical path of the analyte are already known, so that a standard curve line of the analyte with the known concentration can be established out of the body, and then the concentration of the analyte or the presence or absence of the analyte can be calculated. In this embodiment, the implantable inspecting device P uses physical optical measurement to be its inspecting method, and the implantable inspecting device P can be driven by the wireless power supply method, so that the implantable inspecting device P can be implanted in the human body for a long term (such as one, three, six, nine, twelve, twenty-four, or thirty-six months, or even is permanent). On the contrary, the conventional technical uses electrochemistry method or chemical method to inspect, the material needs to be replaced or the device needs to be taken out and implanted into the human body again due to the broken material, and the user needs to undergo multiple surgeries.

In some embodiments, the implantable inspecting device P includes two or more light sources 200, and the wavelengths of these light sources 200 are the same or different.

Referring to Figs. 2 and 3, the wireless transmitting module 600 is electrically connected to the light source 200 and/or the sensor 500, so that the reading device R can be connected to the implantable inspecting device P in a wireless manner. In particular, when the reading device R is adjacent to the implantable inspecting device P, the data that inspected by the sensor 500 after it receives the light can be transmitted to the reading device R through the wireless transmitting module 600 in a wireless manner. Moreover, the reading device R can control the light source 200, switch the wavelength of the light source, adjust the pulse width modulation (PWM), or let the light source 200 emit the light or stop emitting the light through the wireless transmitting module 600.

In this embodiment, the implantable inspecting device P further includes the membrane member 700. The membrane member 700 surrounds the hollow carrier 100 and affixed thereto, and covers or overlays at least some of the cavity portions 120. The membrane member 700 admits the liquid to pass, and can reduce the tissue of the human body from proliferating to the position between the light source 200 and the sensor 500 and influencing the moving path of the light. Moreover, the membrane member 700 can include degradable material or hydrophilic non-degradable material, so as to prevent the human body from the rejection reaction. For example, the membrane member 700 can include calcium carbonate, tricalcium phosphate (TCP), calcium sulfate, starch, cellulose, chitin, collagen, gelatin, hyaluronic acid (HA), polyhydroxyalkanoates (PHA), polycaprolactone (PCL), poly glycolic acid (PGA), polyactic acid (PLA), nylon, polytetrafluoroethylene (PTFE), or expanded polytetrafluoroethylene (EPTFE).

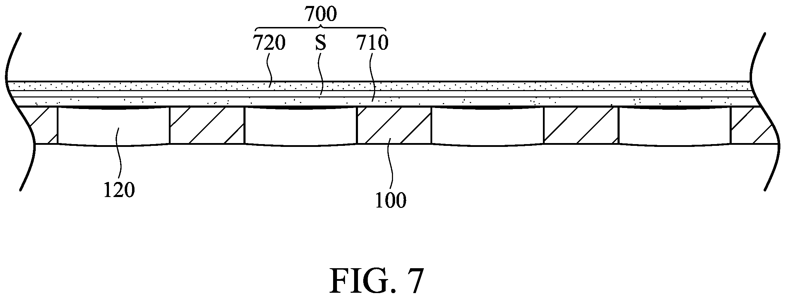

Referring to Fig. 7, in some embodiments, the membrane member 700 can be a single layer of polymer or a composite layer. The composite layer can include a main body S, a retardant layer 710, and a proliferation layer 720, wherein the retardant layer 710 is disposed between the proliferation layer 720 and the hollow carrier 100. The retardant layer 710 is configured to retard the tissue of the human body to proliferate, so that the protein will not attach to the membrane member 700 to influence the inspection. For example, the retardant layer 710 can include paclitaxel (PTX). The proliferation layer 720 is configured to facilitate the tissue of the human body to proliferate and fibrotic scar. For example, the proliferation layer 720 can include collagen, glycosaminoglycans (GAGs), transforming growth factor beta (TGF-β), connective tissue growth factor (CTGF), or interleukin 4 (IL-4). The retardant layer 710 and the proliferation layer 720 of the membrane member 700 can be formed by dipping, coating, or spray coating.

As shown in Figs. 2 and 3, the sealing member 800 can seal the cavity portions 120 on the opposite ends of the hollow carrier 100 or the cavity portion 120 on at least one end of the hollow carrier 100. The function of the sealing member 800 is similar to the membrane member 700. The sealing member 800 is also configured to prevent the excessive tissue of human body from proliferating to the position between the light source 200 and the sensor 500. Therefore, in some embodiments, the membrane member 700 and the sealing member 800 can be integrally formed in one piece. In other words, the membrane member 700 can extend to the end(s) of the hollow carrier 100 to cover the cavity portion(s) 120 on the end(s) of the hollow carrier 100.

In some embodiments, in order to let the liquid entering into the accommodating space 110 of the hollow carrier 100 adequately or rapidly, the membrane member 700 and/or the sealing member 800 can be omitted from the implantable inspecting device P. It should be noted that, in general, the cell, the fiber, or the tissue of the human body is transparent, so that even if they proliferate into the accommodating space 110 of the implantable inspecting device P, the inspecting of the sensor 500 is not significantly influenced.

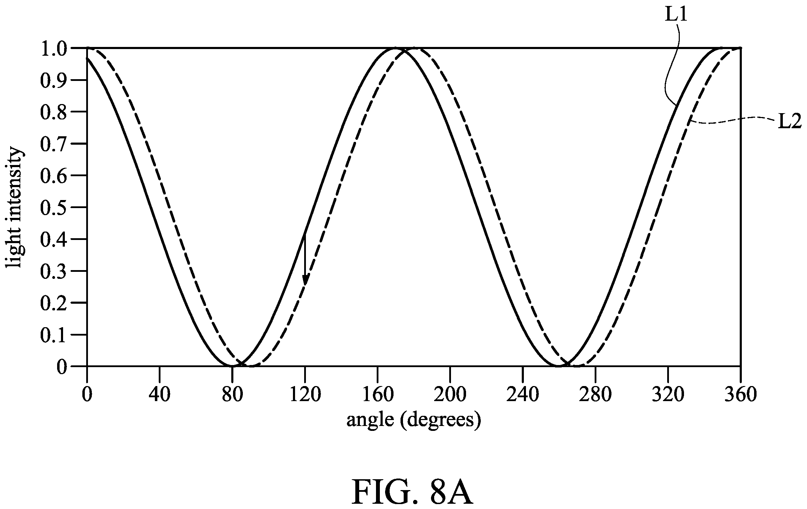

Referring to Fig. 8A, it simulates an example of the data measured by the implantable inspecting device P. The horizontal axis is the optical rotation angle, the vertical axis is the light intensity, the line L1 is the measurement of a standard product with the known concentration and optical activity after it is rotated by the first polarizing member, and line L2 is the measurement of the analyte. The sensor can read the sine wave signal of the analyte, and calculate the concentration of the analyte by comparing the variation of the light intensity (as shown by the arrow).

In order to enhance the resolution for the concentration of the analyte, the LED light source can use PWM control technology to generate a carrier wave with a fixed frequency (such as a square wave or a sine wave) in a flicker frequency greater than 200 Hz, and compare the variation of the light intensity with the waveform passing through the analyte. With the calculation of the phase-locked loop, the concentration of the analyte can be calculated.

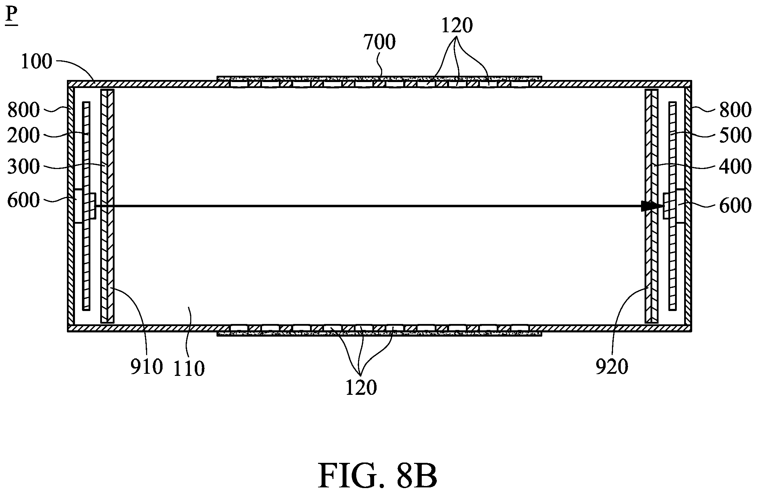

Referring to Fig. 8B, in another embodiment of the invention, the implantable inspecting device P further includes a first light valve module 910. The first light valve module 910 is disposed between the first polarizing member 300 and the second polarizing member 400, and attached to or adjacent to the first polarizing member 300. The first light valve module 910 can change the moving direction of the light from the light source by its own, or drive the first polarizing member 300 to rotate with the first light valve module 910 to let the light emit in different optical rotation angle. For example, the first light valve module 910 can be a faraday rotator, a liquid-crystal module, or a motor-driven mechanical rotator. Owing to the rotation of the first light valve module 910, the sensor 500 can obtain a standard curve line of the analyte with the known concentration in a fixed optical rotation angle (such as the optical rotation angle of the analyte), and the curve line is a sine wave (the horizontal axis is the observation time, and the vertical axis is the variety of the light intensity). In order to enhance the resolution for the concentration of the analyte, the LED light source can use PWM control technology to generate a carrier wave with a fixed frequency (such as a square wave or a sine wave) in a flicker frequency greater than 200 Hz, and compare the variation of the light intensity with the waveform passing through the analyte. With the calculation of the phase-locked loop, the concentration of the analyte or the presence or absence of the analyte can be calculated.

Furthermore, the implantable inspecting device P further includes a second light valve module 920. Similarly, the second light valve module 920 can be disposed between the first polarizing member 300 and the second polarizing member 400, and attached to the second polarizing member 400. The second light valve 920 can rotate alone or drive the second polarizing member 400 to rotate, so that the sensor 500 can obtain periodic data, and the accuracy of the inspecting can be increased.

Similarly, the second light valve module 920 can be a faraday rotator, a liquid-crystal module, or a motor-driven mechanical rotator.

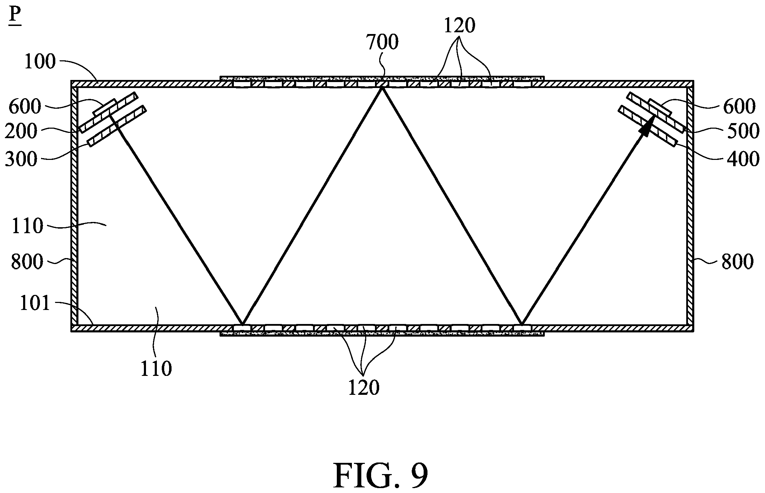

Referring to Fig. 9, in another embodiment, the inner wall 101 of the hollow carrier 100 can be the reflecting surface, and the light from the light source 200 can be reflected several times and received by the sensor 500.

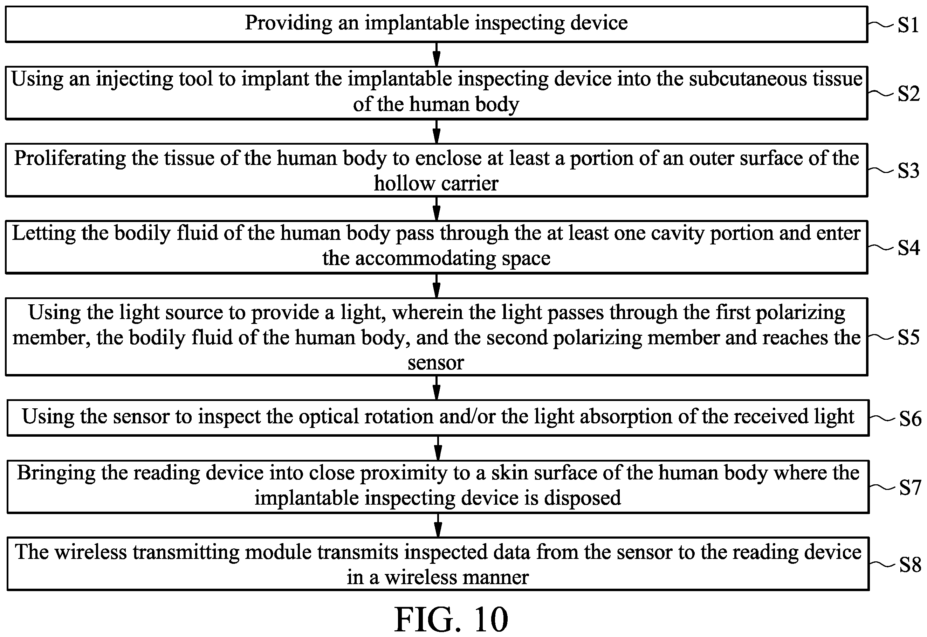

The method for inspecting the analyte in the liquid by using the aforementioned inspecting system is discussed below. In the following discussion, the liquid takes the bodily fluid of the human body B as an example. Referring to Fig. 10 and the aforementioned figures accordingly, first, an implantable inspecting device P can be provided (step S1). The implantable inspecting device P can be any one of the implantable inspecting device P shown in Figs. 2, 8B, and 9, for example. The hollow carrier 100 can include plastic, metal, glass, alloy, or a combination thereof. In some embodiments, the hollow carrier 100 of the implantable inspecting device P can include shape memory alloy, and the appearance thereof can be changed according to the temperature. Therefore, when the implantable inspecting device P is not implanted into the human body B, the volume of the hollow carrier 100 can be less than the status shown in Figs. 5A-6C. Preferably, the length is 2-40mm, the width is 1-10mm, and the accommodating space 110 is close.

Subsequently, the implantable inspecting device P can be subcutaneously implanted into the human body B by using an injecting tool (step S2). For example, the injecting tool can be a syringe, and the subcutaneously implanted position can be the subcutaneous tissue, the connective tissue, the muscle tissue, or the blood tissue. It should be noted that, in the embodiment in that the implantable inspecting device P including shape memory alloy, when the implantable inspecting device P is implanted into the human body B, the hollow carrier 100 is deformed and expanded to the status shown in Figs. 5A-6C due to the temperature of the human body B, and the cavity portions 120 can be therefore formed. Moreover, since the membrane member 700 is flexible, when the hollow carrier 100 is deformed, the membrane member 700 surrounding the hollow carrier 100 can be deformed simultaneously and attached on the outer surface of the hollow carrier 100.

Secondly, the tissue of the human body B can proliferate and enclose at least a portion of the outer surface of the hollow carrier 100 (step S3). Thus, the implantable inspecting device P can be steadily embedded in a fixed position of the human body B. After the implantable inspecting device P is embedded in the human body B, the liquid with the analyte (i.e. the bodily fluid of human body B) can pass the cavity portions 120 on the hollow carrier 100 and enter the accommodating space 110 (step S4).

Then, the light source 200 of the implantable inspecting device P can provide the light, and the light can pass the first polarizing member 300, the liquid with the analyte, and the second polarizing member 400, and reach the sensor 500 (step S5). The sensor 500 can inspect the optical rotation and/or the light absorption of the received light (step S6).

Finally, the reading device R can be close or adjacent to the skin surface of the human body B where the implantable inspecting device P is disposed (step S7), and the sensor 500 can transmit the inspected data to the reading device R through the wireless transmitting module 600 in a wireless manner (step S8). The reading device R can convert the data to the concentration, so that the user can easily read it.

In some embodiments, the step of converting the data can be performed in the sensor 500, and the sensor 500 can transmit the converted result to the reading device R. Moreover, the reading device R can be disposed in a strap, a watch band, or an arm sleeve.

In some embodiments, the wireless transmitting module 600 of the implantable inspecting device P can include charging coil, so that when the reading device R is close or adjacent to the skin surface of the human body B where the implantable inspecting device P is disposed, the reading device R can provide power to the implantable inspecting device P in a wireless manner.

In summary, an implantable inspecting device for inspecting an analyte in a liquid is provided, including a hollow carrier, a light source, a first polarizing member, a second polarizing member, and a sensor. The hollow carrier includes an accommodating space and at least one cavity portion communicated with the accommodating space. The light source, the first polarizing member, the second polarizing member, and the sensor are disposed in the accommodating space. The light from the light source passes through the first polarizing member, the liquid, and the second polarizing member to reach the sensor.

Although some embodiments of the present disclosure and their advantages have been described in detail, it should be understood that various changes, substitutions and alterations can be made herein without departing from the spirit and scope of the disclosure as defined by the appended claims. For example, it will be readily understood by those skilled in the art that many of the features, functions, processes, and materials described herein may be varied while remaining within the scope of the present disclosure. Moreover, the scope of the present application is not intended to be limited to the particular embodiments of the process, machine, manufacture, compositions of matter, means, methods and steps described in the specification. As one of ordinary skill in the art will readily appreciate from the disclosure of the present disclosure, processes, machines, manufacture, compositions of matter, means, methods, or steps, presently existing or later to be developed, that perform substantially the same function or achieve substantially the same result as the corresponding embodiments described herein may be utilized according to the present disclosure. Accordingly, the appended claims are intended to include within their scope such processes, machines, manufacture, compositions of matter, means, methods, or steps. Moreover, the scope of the appended claims should be accorded the broadest interpretation so as to encompass all such modifications and similar arrangements.

While the invention has been described by way of example and in terms of preferred embodiment, it should be understood that the invention is not limited thereto. On the contrary, it is intended to cover various modifications and similar arrangements (as would be apparent to those skilled in the art). Therefore, the scope of the appended claims should be accorded the broadest interpretation to encompass all such modifications and similar arrangements.

**Claims**

1. An implantable inspecting device for inspecting an analyte in a liquid, comprising:

   a hollow carrier, comprising an accommodating space and at least one cavity portion communicated with the accommodating space;
   a light source, disposed in the accommodating space;
   a first polarizing member, disposed in the accommodating space;
   a second polarizing member, disposed in the accommodating space; and
   a sensor, disposed in the accommodating space, wherein light from the light source passes through the first polarizing member, the liquid, and the second polarizing member and reaches the sensor.

2. The implantable inspecting device as claimed in claim 1, wherein the hollow carrier comprises plastic, metal, glass, alloy, or a combination thereof.

3. The implantable inspecting device as claimed in claim 1, wherein the implantable inspecting device further comprises a first light valve module disposed in the accommodating space, and the first light valve module is a faraday rotator, a liquid-crystal module, or a motor-driven mechanical rotator.

4. The implantable inspecting device as claimed in claim 3, wherein the implantable inspecting device further comprises a second light valve module, and the light from the light source passes through the first polarizing member, the first light valve module, the liquid, the second light valve module, and the second polarizing member and reaches the sensor.

5. The implantable inspecting device as claimed in claim 1, wherein the implantable inspecting device further comprises a sealing member, and the hollow carrier comprises a plurality of cavity portions, wherein the sealing member is connected to the hollow carrier and covers at least some of the cavity portions.

6. The implantable inspecting device as claimed in claim 1, wherein the hollow carrier has a pillar structure, a ball structure, a capsule-shaped structure, or a fusiform-shaped structure.

7. The implantable inspecting device as claimed in claim 1, wherein the at least one cavity portion has a circular structure, a polygonal structure, a longitudinal structure, a spiral structure, or an irregular shape structure.

8. The implantable inspecting device as claimed in claim 1, wherein the entire area of the least one cavity portion occupies 30% - 70% of the surface area of a space in which the hollow carrier 100 is disposed.

9. The implantable inspecting device as claimed in claim 1, wherein the inspecting device further comprises a wireless transmitting module electrically connected to the sensor and/or the light source.

10. The implantable inspecting device as claimed in claim 1, wherein the inspecting device further comprises a membrane member, and the hollow carrier comprises a plurality of cavity portions, wherein the membrane member is disposed on the hollow carrier and covers at least some of the cavity portions.

11. The implantable inspecting device as claimed in claim 10, wherein the membrane member comprises degradable material or hydrophilic non-degradable material.

12. The implantable inspecting device as claimed in claim 10, wherein the membrane member comprises a retardant layer and a proliferation layer, and the retardant layer is disposed between the proliferation layer and the hollow carrier.

13. The implantable inspecting device as claimed in claim 1, wherein the range of the wavelength of the light source is between the wavelength of the near infrared and the wavelength of the blue light.

14. The implantable inspecting device as claimed in claim 1, wherein the light source, the first polarizing member, the second polarizing member, and the sensor are arranged along a longitudinal axis of the hollow carrier in sequence.

15. An inspecting system for inspecting an analyte in a liquid, comprising:
    an implantable inspecting device, comprising:

    a hollow carrier, comprising an accommodating space and at least one cavity portion communicated with the accommodating space;
    a light source, disposed in the accommodating space;
    a first polarizing member, disposed in the accommodating space;
    a second polarizing member, disposed in the accommodating space;

    a sensor, disposed in the accommodating space, wherein light from the light source passes through the first polarizing member, the liquid, and the second polarizing member and reaches the sensor; and
    a wireless transmitting module, electrically connected to the sensor; and
    a reading device, wherein when the reading device is adjacent to the inspecting device, the wireless transmitting module transmits inspected data from the sensor to the reading device in a wireless manner.

16. An inspecting method for an implantable inspecting device, comprising:

    providing an implantable inspecting device, wherein the implantable inspecting device comprises a hollow carrier, a light source, a first polarizing member, a second polarizing member, and a sensor, the hollow carrier comprises an accommodating space and at least one cavity portion communicated with the accommodating space, and the light source, the first polarizing member, the second polarizing member, and the sensor are disposed in the accommodating space;
    letting a liquid pass through the at least one cavity portion and enter the accommodating space;
    using the light source to provide a light, wherein the light passes through the first polarizing member, the liquid, and the second polarizing member and reaches the sensor; and
    using the sensor to inspect the optical rotation and/or the light absorption of the light, so as to inspect the analyte.

17. An inspecting method for inspecting an analyte in a bodily fluid of a human body, comprising:

    providing an implantable inspecting device, wherein the implantable inspecting device comprises a hollow carrier, a light source, a first polarizing member, a second polarizing member, and a sensor, the hollow carrier comprises an accommodating space and at least one cavity portion communicated with the accommodating space, and the light source, the first polarizing member, the second polarizing member, and the sensor are disposed in the accommodating space;
    using an injecting tool to implant the implantable inspecting device into the human body;
    proliferating the tissue of the human body to enclose at least a portion of an outer surface of the hollow carrier;
    letting the bodily fluid of the human body pass through the at least one cavity portion and enter

**EP 4 011 276 A1**

the accommodating space;
using the light source to provide a light, wherein the light passes through the first polarizing member, the bodily fluid of the human body, and the second polarizing member and reaches the sensor; and
using the sensor to inspect the optical rotation and/or the light absorption of the light, so as to inspect the analyte.

18. The inspecting method as claimed in claim 17, wherein in the step of implanting the inspecting device into the human body, the inspecting device is implanted into a subcutaneous tissue, a connective tissue, a muscle tissue, or a blood tissue.

19. The inspecting method as claimed in claim 18, wherein the implantable inspecting device further comprises a wireless transmitting module electrically connected to the sensor, and the inspecting method further comprises:

providing a reading device;
bringing the reading device into close proximity to a skin surface of the human body adjacent to the implantable inspecting device; and
using the wireless transmitting module to transmit inspected data from the sensor to the reading device in a wireless manner.

20. The inspecting method as claimed in claim 19, wherein the inspecting method further comprises using the reading device to provide power to the implantable inspecting device in a wireless manner.

FIG. 1

FIG. 2

FIG. 3

EP 4 011 276 A1

FIG. 4

100

110

120

120

120

**FIG. 5A**

100

120

120

120

**FIG. 5B**

100

120                    120

110

FIG. 5C

EP 4 011 276 A1

FIG. 5D

FIG. 5E

FIG. 6A

FIG. 6B

FIG. 6C

EP 4 011 276 A1

FIG. 7

EP 4 011 276 A1

FIG. 8A

FIG. 8B

FIG. 9

EP 4 011 276 A1

S1 — Providing an implantable inspecting device

↓

S2 — Using an injecting tool to implant the implantable inspecting device into the subcutaneous tissue of the human body

↓

S3 — Proliferating the tissue of the human body to enclose at least a portion of an outer surface of the hollow carrier

↓

S4 — Letting the bodily fluid of the human body pass through the at least one cavity portion and enter the accommodating space

↓

S5 — Using the light source to provide a light, wherein the light passes through the first polarizing member, the bodily fluid of the human body, and the second polarizing member and reaches the sensor

↓

S6 — Using the sensor to inspect the optical rotation and/or the light absorption of the received light

↓

S7 — Bringing the reading device into close proximity to a skin surface of the human body where the implantable inspecting device is disposed

↓

S8 — The wireless transmitting module transmits inspected data from the sensor to the reading device in a wireless manner

FIG. 10

## INTERNATIONAL SEARCH REPORT

International application No.

**PCT/CN2019/100080**

**A. CLASSIFICATION OF SUBJECT MATTER**

A61B 5/00(2006.01)i; A61B 5/145(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNABS; CNTXT; CNKI; VEN; USTXT; EPTXT; WOTXT: 精能医学, 血糖, 葡萄糖, 体液, 注入, 植入, 皮下, 检测, 感测, 监测, 偏振, 偏光, glucose, detect+, monitor+, polariz+, implant+, inject+

**C. DOCUMENTS CONSIDERED TO BE RELEVANT**

| Category* | Citation of document, with indication, where appropriate, of the relevant passages | Relevant to claim No. |
|---|---|---|
| X | CN 102333478 A (GLUSENSE LTD.) 25 January 2012 (2012-01-25) <br> claims 1, 20-22, description, paragraphs [0012], [0316]-[0334], [0354]-[0510], figure 1 | 1-20 |
| A | CN 104323781 A (SHANGHAI HICLING ELECTRONIC SCIENCE & TECHNOLOGY CO., LTD.) 04 February 2015 (2015-02-04) <br> entire document | 1-20 |
| A | CN 107029353 A (GLUSENSE LTD) 11 August 2017 (2017-08-11) <br> entire document | 1-20 |
| A | CN 107898439 A (DAI, Yuguo) 13 April 2018 (2018-04-13) <br> entire document | 1-20 |
| A | CN 201147318 Y (RESEARCH INSTITUTE OF FIELD SURGERY OF THIRD MILITARY MEDICAL UNIVERSITY OF PLA) 12 November 2008 (2008-11-12) <br> entire document | 1-20 |

☐ Further documents are listed in the continuation of Box C.  ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **24 March 2020** | **23 April 2020** |

| Name and mailing address of the ISA/CN | Authorized officer |
|---|---|
| **China National Intellectual Property Administration (ISA/CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2019/100080**

| Patent document cited in search report | | | Publication date (day/month/year) | Patent family member(s) | | | Publication date (day/month/year) |
|---|---|---|---|---|---|---|---|
| CN | 102333478 | A | 25 January 2012 | EP | 2378954 | A1 | 26 October 2011 |
| | | | | WO | 2010073249 | A1 | 01 July 2010 |
| | | | | US | 2012059232 | A1 | 08 March 2012 |
| | | | | CN | 102333478 | B | 10 December 2014 |
| | | | | EP | 2378954 | A4 | 15 May 2013 |
| CN | 104323781 | A | 04 February 2015 | None | | | |
| CN | 107029353 | A | 11 August 2017 | US | 2017100598 | A1 | 13 April 2017 |
| CN | 107898439 | A | 13 April 2018 | None | | | |
| CN | 201147318 | Y | 12 November 2008 | None | | | |

Form PCT/ISA/210 (patent family annex) (January 2015)